# EUROPEAN PATENT APPLICATION

(11) **EP 1 097 717 A1**
(43) Date of publication of application: **09.05.2001**
(21) Application number: 99929785.6
(22) Date of filing: 09.07.1999
(51) Int. Cl.: A61K 38/17

(54) **REMEDIES FOR APOPTOSIS-ASSOCIATED DISEASES**

(30) Priority: 10.07.1998 JP 21029798; 22.09.1998 JP 28476098
(71) Applicant: MEIJI MILK PRODUCTS COMPANY LIMITED, Tokyo 104-0031 (JP); Muramatsu, Takashi, Nagoya-shi, Aichi 468-0021 (JP)
(72) Inventor: MURAMATSU, Takashi, Nagoya-shi, Aichi 468-0011 (JP); IKEMATSU, Shinya, Meiji Milk Products Co., Ltd., Odawara-shi, Kanagawa 250-0862 (JP); YOSHIDA, Yoshihiro, Kagoshima-shi, Kagoshima 890-0032 (JP); KADOMATSU, Kenji, Lions-Mansion Nakahira 101, Nagoya-shi, Aichi 468-0014 (JP); ODA, Munehiro, Meiji Milk Products Co., Ltd., Odawara-shi, Kanagawa 250-0862 (JP); SAKUMA, Sadatoshi, Meiji Milk Products Co., Ltd., Odawara-shi, Kanagawa 250-0862 (JP); ASHIDA, Kin-ya, Odawara-shi, Kanagawa 250-0862 (JP); KINO, Kohsuke, Odawara-shi, Kanagawa 250-0862 (JP)
(74) Representative: Grünecker, Kinkeldey, Stockmair & Schwanhäusser Anwaltssozietät
(86) International application number: JP9903740
(87) International publication number: WO0002578

(57) **Abstract**

Proteins belonging to the MK family can suppress apoptosis induced by various stimuli such as carcinostatics, ultraviolet light and radiation, ischemic stresses, etc. Based on this finding, the invention provides novel agents for treating or preventing various diseases attributed to apoptosis such as cerebropathy, cardiopathy, nephropathy, neuropathy, or hepatopathy, etc., comprising proteins belonging to the MK family as an effective ingredient.

## Description

### Technical field

The present invention relates to agents for suppressing apoptosis, comprising a protein belonging to the midkine family as an effective ingredient, and agents for treating or preventing apoptosis-related disorders.

### Background Art

Apoptosis was discovered due to the morphological differences with necrosis (Kerr, J. F. R. et al.: Brit. J. Cancer, 26: 239-257, 1972). In necrosis, the whole cell as well as the mitochondria gradually enlarge and cytoplasmic changes proceed. Finally, the cell membrane erupts causing cytolysis, generally accompanies inflammation. In contrast, in apoptosis, the first change occurs in the nucleus, and the nucleus and cell both shrink. In the cytoplasm, organelles such as mitochondra remain normal. Ultimately, apoptotic bodies form, and the cell is completely phagocytosed by macrophages or adjacent phagocytes. No inflammation is observed.

Necrosis is a "pathological cell death" in which a group of cells damaged by pathological factors such as burns dies all at once, while apoptosis is a "physiological cell death" caused by not only pathological factors, but also various physiological factors such as development, immune or hormone actions. Apoptosis sporadically occurs at random in terms of time and site and plays an important role as a cell death essential for biological phenomena.

Apoptosis differs from necrosis not only morphologically but also functionally, playing an important function in cellular dynamics in tissues together with its opposite, the cell division. Therefore, apoptotic abnormalities are deeply related to the onset of various diseases. Disorders related to lowered apoptosis include canders, autoimmune disorders, viral infections, etc. Enhanced apoptosis may cause acquired immune deficiency syndrome (AIDS), Alzheimer's disease, amyotrophic lateral sclerosis, pigmentary retinopathy, cerebellar degeneration, aplastic anemia, myocardial infarction, apoplexy, reperfusion injury, alcoholic hepatopathy, parodontal diseases, etc. (Craig, B. T. Science, 267: 1456-1462, 1995). Since 1993, reports supporting apoptosis in regional cerebral ischemia model (rat) have been seen following other apoptosis research (Linnik, M. D. et al.: Stroke, 24: 2002-2009, 1993; Li, Y. et al.: J. Cereb. Blood Flow. Metab., 15: 389-379, 1995; Linnik, M. D. et al.: Mol. Brain Res., 32: 116-124, 1995; Islam, N. et al.: Neurosci. Lett., 188: 159-162, 1995; Soriano, M. A. et al.: Neuroreport, 7: 425-428, 1996; Charriaut-Marlangue, C. et al,: J. Cereb. Blood Flow. Metab., 16: 186-194, 1996; Du, C. et al.: Cereb. Blood flow. Metab., 16: 195-201, 1996).

Recently, treatment and prevention of apoptosis-related diseases have been attempted by inducing or suppressing apoptosis. For example, known methods include the method for preventing or delaying apoptosis by administering therapeutically effective and physiologically acceptable dioxopiperazine (WO95/03054), the treatment and prevention for apoptosis-related diseases by inducing or suppressing apoptosis by apoptosis-related genes (WO96/12017), the method for treating or preventing diseases or morbidity leading to apoptosis by enhancing activity of Bcl-2 in cells (WO94/27426), the method for treating the patients' Fas-mediated cell death by administering a therapeutically effective amount of a novel Fas protein (WO95/13701), the pharmaceutical composition for inhibiting Fas ligand-mediated apoptosis by a monoclonal antibody specifically binding to human Fas antigen (WO95/10640), and such.

### Disclosure of the Invention

No effective method for treating numerous disorders causing abnormal apoptotic cell death, including all of the above-mentioned disorders, was available. An objective of the present invention is to provide a method for treating and preventing apoptosis-related diseases by suppressing the above-mentioned apoptosis. The present inventors discovered that midkine (MK), a heparin-binding growth and differentiation factor, which has various biological activities such as elongation of neurite, survival of neurocytes, and activation of fibrinolytic system in blood vessel endothelial cells, can suppress apoptosis caused by carcinostatics and ischemic stresses, thereby completing the invention.

The present invention relates to agents for suppressing apoptosis, comprising a protein belonging to the MK family as an effective ingredient, more specifically, relates to:
(1) an agent for suppressing apoptosis, comprising a protein belonging to the MK family as an effective ingredient;
(2) the agent for suppressing apoptosis of (1), wherein the protein belonging to the MK family is midkine;
(3) an agent for treating or preventing apoptosis-related diseases, comprising a protein belonging to the MK family as an effective ingredient;
(4) the agent for treating or preventing apoptosis-related diseases of (3), wherein the apoptosis-related disease is cardiopathy;
(5) the agent for treating or preventing apoptosis-related diseases of (3), wherein the apoptosis-related disease is nephropathy;
(6) the agent for treating or preventing apoptosis-related diseases of (3), wherein the apoptosis-related disease is hepatopathy;
(7) the agent for treating or preventing apoptosis-related diseases of (3), wherein the apoptosis-related disease is a neurodegenerative disease;
(8) the agent for treating or preventing apoptosis-related diseases of (3) to (7), wherein the protein belonging to the MK family is midkine; and,
(9) a Bcl-2 enhancer comprising a protein belonging to the MK family as an effective ingredient.

This invention also relates to the following method using proteins, belonging to the MK family.

The present invention relates to a method for suppressing apoptosis by administering a protein belonging to the MK family, a method for treating or preventing apoptosis-related diseases by administering a protein belonging to the MK family, and a method for enhancing Bcl-2 expression by administering a protein belonging to the MK family.

Further, this invention relates to the following use of proteins belonging to the MK family. Specifically, the present invention relates to the use of a protein belonging to the MK family for preparing a pharmaceutical composition for suppressing apoptosis, the use of a protein belonging to the MK family for preparing a pharmaceutical composition for treating or preventing apoptosis-related diseases, and the use of a protein belonging to the MK family for preparing a pharmaceutical composition for enhancing Bcl-2.

The current invention is based on the findings of the inventors that MK, a heparin-binding growth and differentiation factor, can suppress apoptosis caused by carcinostatics and ischemic stresses. Therefore, the first aspect of this invention is to suppress apoptosis by a protein belonging to the MK family.

Midkine was discovered as a product of a gene whose expression was induced at the early stage of the differentiation and induction processes of embryonic tumor cells by retinoic acid (Kadomatsu, K. et al.: Biochem. Biophys. RES. Commun., 151: 1312-1318, 1988). Pleiotrophin (PTN) was discovered in the brain of a newborn rat as a heparin-binding protein with neurite elongation ability (Rauvala, H.: EMBO J., 8: 2933-2941, 1989). Midkine and Pleiotrophin are heparin-binding proteins that control cell proliferation, survival and differentiation in the development processes (Tomomura, M. et al., J. Biol. Chem., 265: 10765-10770; Li, Y. et al.: Science 250: 1690-1694, 1990; Rauvala, H.: EMBO J., 8: 2933-2941, 1989; Wellstein, A. et al.: J. Biol. Chem., 267: 2582-2587, 1992). Mature MK and PTN are composed of 123 and 136 amino acids rich in basic amino acids and cysteins, respectively, exhibiting 50% homology to each other (Tomomura, M. et al.: J. Biol. Chem., 265: 10765-10770; Kuo, M. et al.: Biol. Chem., 265: 18749-18752; Tsutsui, J. et al.: Biochem. Biophys. Res. Commun., 176: 792-797, 1991), and form the MK family (Muramatsu, T.: Dev. Growth Differ., 36: 1-8, 1994).

Carcinostatics kill cells by inducing apoptosis (Gunji, H. et al.: Cancer Res., 51: 747-743, 1991; Kaufmann, S. H.: Cancer Res., 49: 5870-5878, 1989). Radiation and ultraviolet light are also known to induce apoptosis (Miura, M., Yamada, T. eds.: Experimental Medicine Supplement "Terminology Library Apoptosis" 1996). It was also demonstrated that delayed neuronal death of brain cells caused by ischemia is also due to apoptosis (J. Neurosci., 19: 4200-4210, 1999).

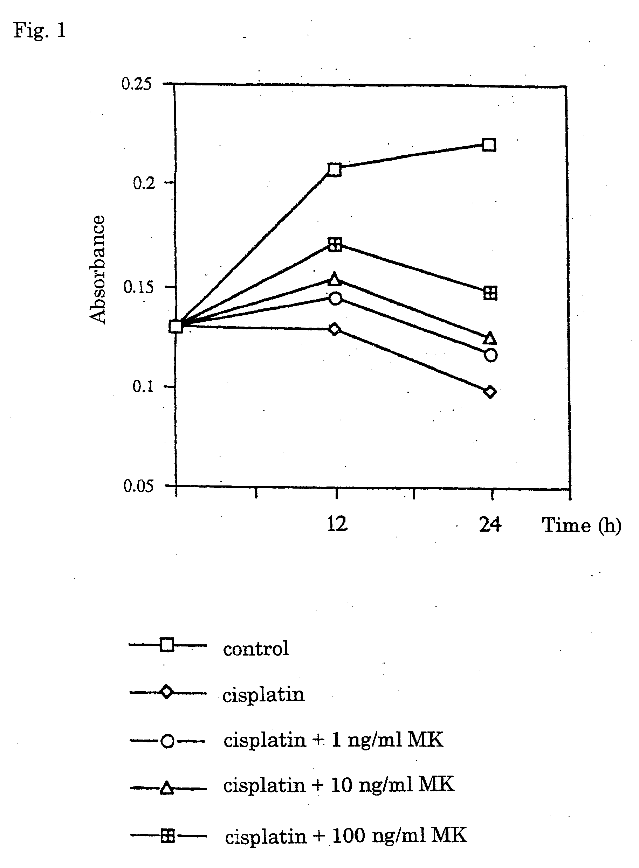

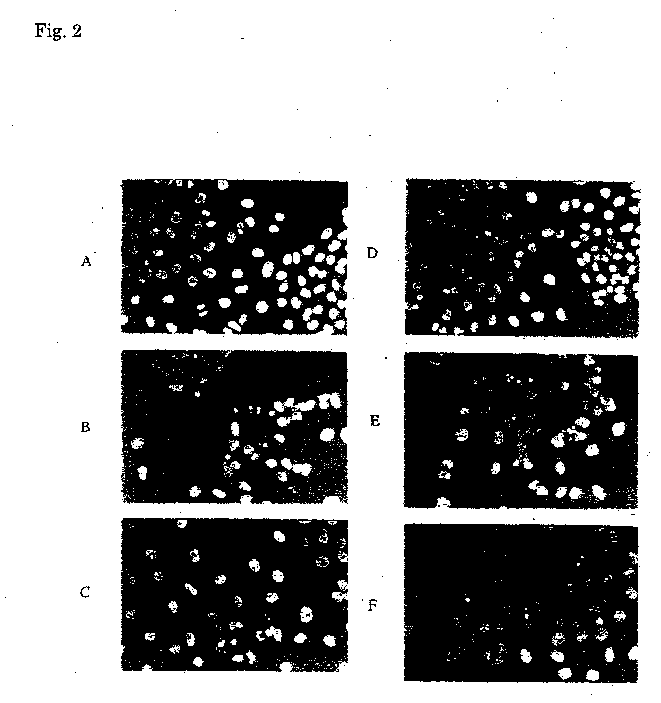

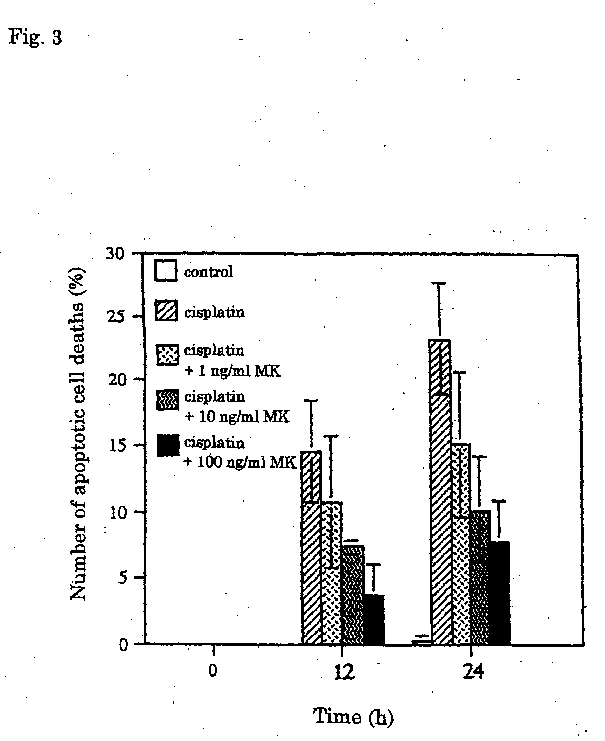

Based on the above background, the present invention will be explained with reference to the following figures and tables. Figure 1 shows the number of live cells (counted by MTT method) in cultured cells treated with a carcinostatic to induce apoptosis. In the MK + carcinostatic-treatment group, the number of live cells increased depending on the concentration of MK, compared with the control group treated only with the carcinostatic. Figure 2 shows the cultured cells treated with the carcinostatic to induce apoptosis and stained with Hoechst33342. Panels B (cultured for 12 hours) and E (cultured for 24 hours) clearly show condensation of chromatin and formation of apoptosis corpuscle specific for apoptosis in the control group treated only with the carcinostatic. In contrast, panels C (cultured for 12 hours), and F (cultured for 24 hours) show that these phenomena were suppressed in the MK + carcinostatic-treatment group. Figure 3 numerically shows the results of Fig. 2. The results demonstrate that the rate of apoptotic cell death decreased depending on the concentration of MK in the MK + carcinostatic-treatment group, compared with the control group treated only with the carcinostatic.

In cultured cell lines, apoptosis is controlled by the cell cycle, similarly to cell proliferation. In the cell cycle, G1 phase has the lowest DNA content (2C) and G2 phase has double of that at G1 phase (4C). At the S phase, the DNA content is between 2C and 4C, depending on the DNA synthesis rate. At the M phase, the DNA content decreases from 4C to 2C during cell division. Therefore, the distribution of cells during cell cycle can be identified by staining cells with a DNA-binding fluorescent pigment (such as propidium iodide, ethidium bromide, etc.) and measuring fluorescence intensity by flow cytometry (FACS) to determine the cellular DNA content. Prior to DNA staining, low molecular weight DNA fragmented by the fixation with 70% ethanol leaks from cells resulting in the detection of apoptotic cells whose DNA content is less than that at G1 phase. The present inventors measured the DNA content of survived cells to determine the distribution of cells at G1, S, and S/M phases. In the presence or absence of a MK, neurocytes were exposed to ultraviolet light and stained with a DNA-binding fluorescent pigment to measure DNA by FACS to analyze the cell cycle. As shown in Table 1, in the presence of MK, the number of cells at G1 phase increased almost tenfold, but decreased a eighth to a forth at S phase, compared to the control group. These results suggest that MK suppresses the induction of cellular apoptosis caused by ultraviolet light or radiation.

Moreover, MK can relieve drug-induced nephropathy and hepatopathy (International Patent Application No. PCT/JP98/01050). The following analyses were conducted to confirm whether MK suppresses apoptosis in uriniferous tubule cells. An apoptotic cell is characterized by the fragmentation of chromatin DNA at a nucleosome (185 bp) level. The amount of the fragmented DNA can be histochemically detected by TdT-mediated dUTP-biotin nick end labeling (TUNEL method). TUNEL method detects DNA by labeling 3'-OH end of DNA with biotin-dUTP.

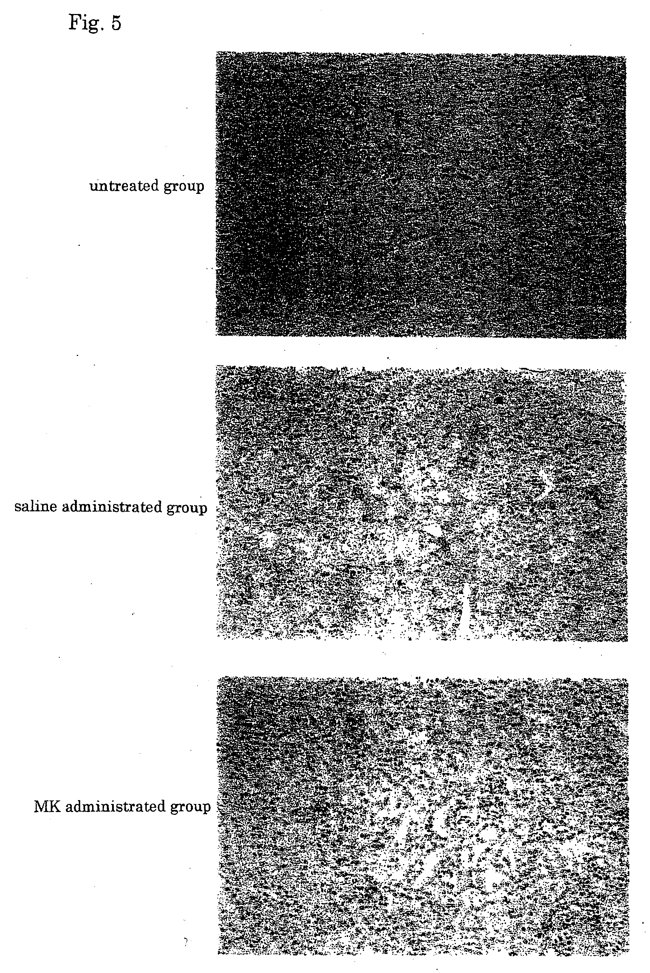

A carcinostatic was administered to MK knockout mice, and paraffin sections of mouse kidneys, which are representative samples showing strong damage were prepared to detect DNA fragmentation by the TUNEL method (In situ Apoptosis Detection Kit; TaKaRa). There were some TUNEL-positive nuclei in the saline administrated group while considerably much less TUNEL-positive nuclei were seen in the MK administration group (Fig. 5). In the untreated normal mice group, no TUNEL-positive nuclei were observed. These results suggest that MK can suppress uriniferous tubule cell apoptosis caused by a carcinostatic.

The present invention demonstrated for the first time that MK could suppress apoptotic cell death caused by various stimuli through *in vitro* and *in vivo* apoptosis-induction experiments in the presence of MK. A person skilled in the art can easily expect that PTN belonging to the MK family can similarly suppress apoptosis and may confirm this hypothesis by conducting an established apoptosis experimental method. Therefore, PTN is also included in this invention.

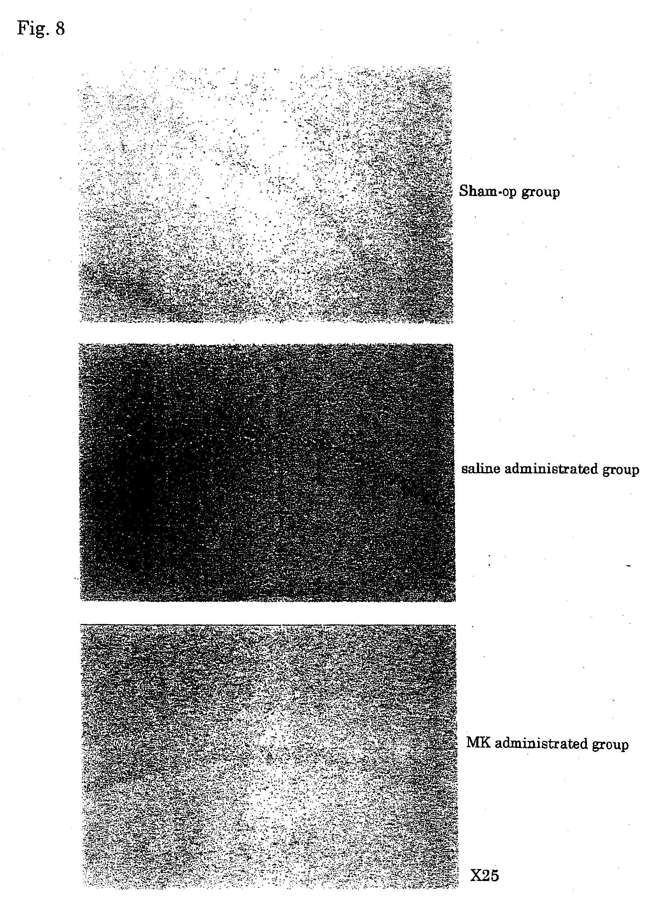

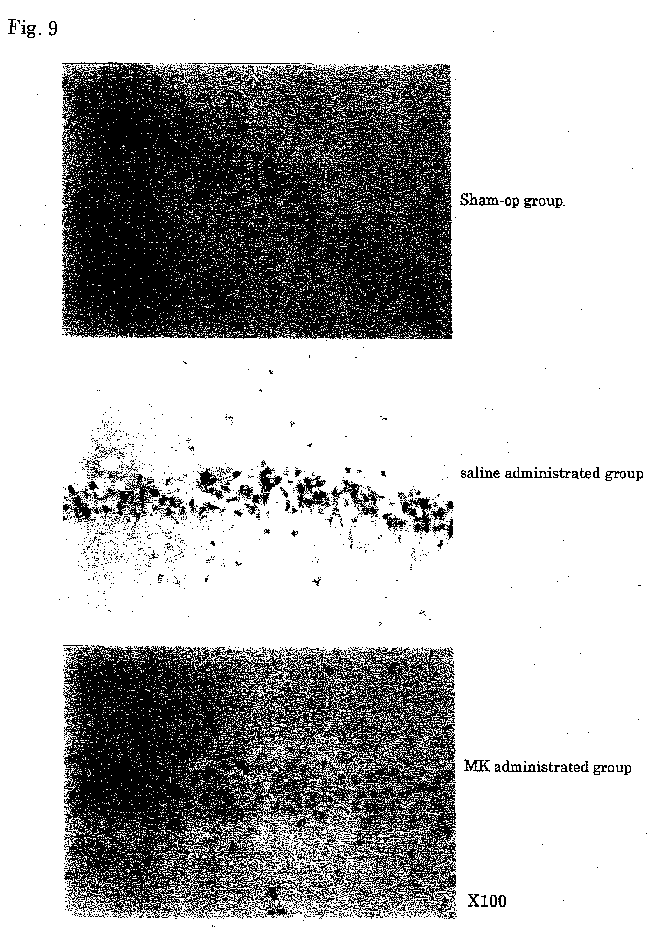

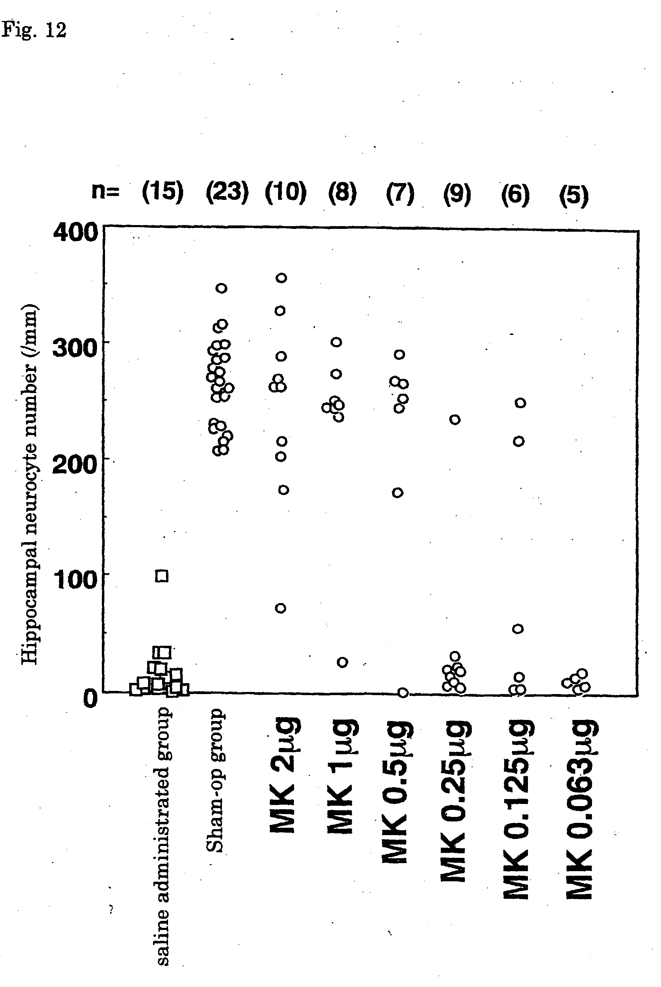

The present invention provides agents effective for relieving or suppressing apoptosis. This invention also includes administration of a MK family protein in an amount therapeutically effective for suppressing or delaying apoptosis. Symptoms of apoptotic suppression *in vivo* includes, the suppression of apoptotic cell death in transient ischemia caused by the decline of normal blood circulation, and the apoptotic cell death suppression in the myocardium, brain, and kidney after reperfusion injury. It is known that apoptosis is a cause of perfusion damage attributed to coronary artery occlusion; severe paralysis caused by or accompanied by spinal cord/head injury; and perfusion damage caused by other damages such as frostbite. The MK family is especially effective for treating these apoptosis-related disorders. The MK family is also effective for treating Indication, which was thought to be treatable by superoxide dismutase (SOD) or antioxidant. Ischemic cellular damage following coronary artery occlusion can lead to acute myocardial infarction (AMI). AMI is known to cause embolus, thrombus or focal necrosis in macroscopic regions. Subsequently, sudden insufficiency of oxygen-rich blood caused by low blood pressure affects heart, brain, spleen, kidneys intestine, lungs and testes. Until recently, infarct-related cell death was thought to be caused directly by ischemia. Now it is known that apoptosis is caused by tissues in ischemic regions, and perfusion of oxygen-rich blood into these regions. Figures 8 and 9 show that apoptosis in hippocampal region was suppressed by administering MK to transient forebrain ischemia model mice. Moreover, Fig. 12 shows delayed neuronalnal death in the hippocampal region caused by apoptosis was suppressed by administering MK to the same ischemia model mice. These results imply that the proteins belonging to the MK family can suppress apoptosis caused by ischemic stresses.

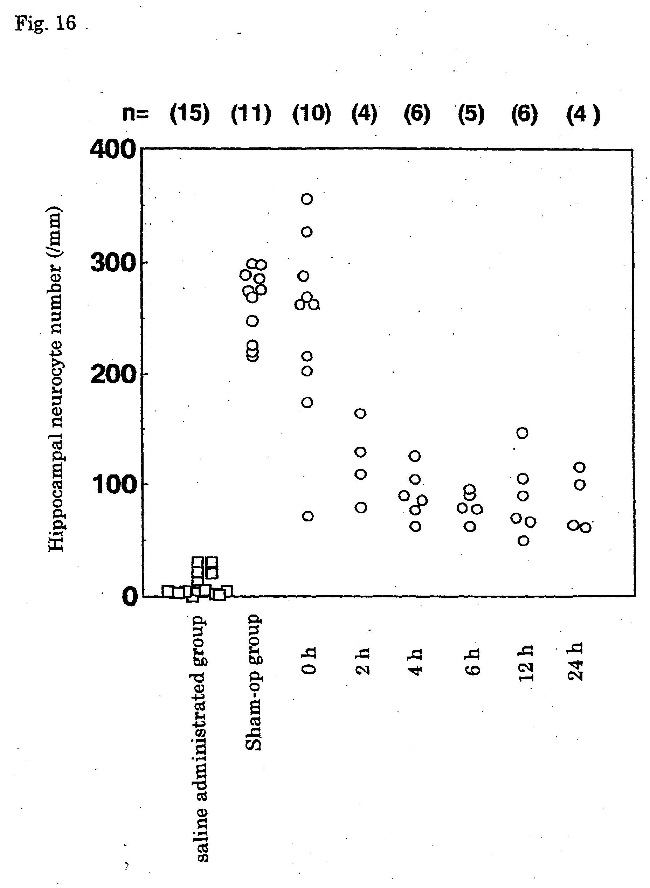

Furthermore, Fig. 16 shows apoptosis was suppressed by administering MK to the ischemia model mice even after ischemia was developed. This elucidates that proteins belonging to the MK family can be effective drugs for treating various disorders attributed to apoptosis.

MK was found to relieve hippocampal delayed cell death following transient forebrain ischemia in the Mongolian gerbils. It has been discovered that NGF and bFGF ameliorate delayed neuronal death in this system (Shigeno, T. et al., J. Neurosci., 11: 2914-2919, 1991; Nakata, N. et al: Brain Res., 605: 354-356, 1993). The dosage of NGF required for protection upon intraventricular injection is 10 µg (Shigeno, T. et al.: J. Neurosci., 11: 2914-2919, 1991), and continuous injection with an osmotic minipump was necessary for bFGF (Nakata, N. et al.: Brain Res., 605: 354-356, 1993). MK measured by this method shows remarkable neurotrophic activity *in vivo*.

Moreover, administration of NGF is effective for rescuing hippocampal neurocytes from delayed neuronal death seven days after development of the ischemic injury, however, not effective 28 days after the injury (Ishimura, H. et al.: Brain Res., 789: 194-200, 1998). The present inventors demonstrated that MK showed a significant activity even at 28 days after the injury. More importantly, MK administered after the injury enhanced survival of neurocytes.

The fact that MK has activity for preventing neurocyte death *in vivo* suggests that MK can be used as drugs for preventing neurocyte death in cerebral infarction and neurodegenerative disorders. Being basic in nature, MK proteins may pass the blood-brain barrier to some extent. Synergystic effects of MK and NGF on promoting the survival of embryonic neurocytes have been observed (Michikawa, M. et al.: J. Neurosci. Res., 35: 530-539, 1993). This hints the possibility of simultaneous administration of MK and other neurotrophic factors.

Therefore, administration of proteins belonging to the MK family at the initiation of acute ischemia, and during or immediately after the circulation of blood rich in oxygen is also effective.

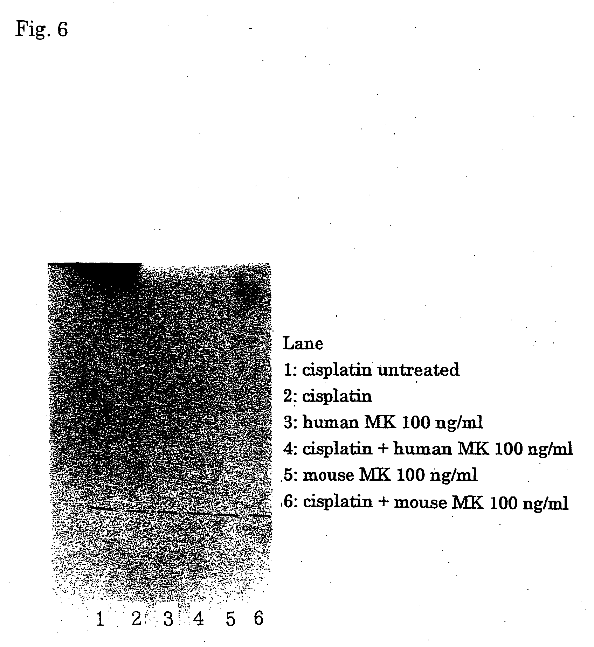

The result of Western blotting of Fig. 6 shows that MK enhanced expression of Bcl-2 in cultured cells. This enhancement was also observed when the cells were treated with a carcinostatic.

After being cloned by Tsujimoto et al (Tsujimoto. Y. et al.: Science, 226: 1097-1099. 1984), Bcl-2 gene is one of the most intensively studied genes in the research field of apoptosis regulation mechanisms Some molecules constituting the family have been discovered from the homology among Bcl-2 coding regions, using the two-hybrid method, etc. studies have focused on mechanisms for controlling apoptosis by the binding to these molecules and intermolecular interaction. Identification of molecules of this family has increased the complexity of the apoptosis regulation mechanism involving Bcl-2, and one has to consider the associations and quantitative balance between each molecule to understand the whole picture.

Apoptosis can be induced by various stimuli, for example, oncogenes such as p53 cancer suppressing gene, c-myc, rae, etc., carcinostatics, ultraviolet and radiation and some kinds of cytokines represented by Fas ligand. Many induction signals finally flow into a common pathway controlled by apoptosis-performing factor caspase and apoptosis-suppressing factor Bcl-2 family (Vaux, D. L. et al.: Nature, 335: 440-442, 1998).

Under these circumstances, the present invention provides agents for enhancing Bcl-2 in cells influenced by diseases or disorders accompanying apoptosis, and agents for decreasing Bcl-2 activity in cancerous or virus-infected cells for enhancing sensitivity of the cells to the treatment.

The present invention utilizes apoptosis suppressing activity of Bcl-2. Targeting physiological mechanisms common for many various diseases is efficient and economical, since there is no need to develop different drugs for each specific disease.

MK and PTN used for implementing this invention can be natural, chemically synthesized, recombinant proteins, etc. The amino acid sequences of MK and PTN are not limited to intact sequences. MK and PTN can be partially modified without destroying their biological functions. Biologically similar genes of MK and PTN can be obtained by a modification such as a deletion, insertion, or substitution of the amino acids in the sequences. Exemplified modification is an alteration of a genetic sequence at a specific site, which produces a chemically equivalent amino acid. Such an alteration should be based on relative similarity among amino acid residues, including hydrophobicity, hydrophilicity, electrical charge, and size. The preferable substitution is within the common knowledge of a person skilled in the art considering these various properties, and includes, without limitation, glycine/alanine; valine/isoleucine/leucine; asparagine/glutamine; aspartic acid/glutamic acid; serine/threonine; lysine/arginine; and phenylalanine/tyrosine.

A fragment having at least one function of the complete protein of MK or PTN is included in this invention. For example, the use of C-terminal half 60-121 or C-terminal half 62-104 at C terminus of MK (Muramatsu, H. et al.: Biochem. Biophys. Res. Commun. 203: 1131-1139, 1994), having neurite elongation ability and heparin-binding site, is included in the techniques of a person skilled in the art. The proteins in this invention include polypeptides.

Moreover, biological or functional equivalents of MK or PTM can be prepared by site-directed mutagenesis.

Types and extent of modification of proteins obtained by expressing the cloned MK or PTN in host cells can be determined mainly by post-translational modification ability of the host cells and modification signals existing in the amino acid sequences of proteins. For example, it is well known that glycosylation does not occur in bacterial cells, such as *E. coli*. Therefore, when glycosylation is desired, the proteins are generally expressed in eukaryotic cells. Yeast or insect cells often perform post-translational glycosylation like mammals. MK or PTN used in the present invention includes these modifications.

Proteins such as MK or PTM are rapidly digested by protease in digestive tracts when they are administered orally. To stabilize MK or PTM *in vivo*, a hybrid MK or hybrid PTM should be prepared by binding it to a water-soluble macromolecule such as polyethylene glycol, or polyvinylpyrrolidone. Hybrid with IL-6 or TNF-α have been prepared, and the function has been enhanced by selecting the most suitable hybrid condition (Teutsumi, Y. et al.: Br. J. Cancer., 74; 1090-1095; Tsutsumi, Y. et al.: J. Control Release, 33: 447-451, 1995).

Apoptosis-related disorders treated or prevented by the agents of the invention are not particularly limited as long as they are related to apoptosis. For example, the agents can be applied to glomerulonephritis such as acute glomerulonephritis, chronic glomerulonephritis, diabetic nephritis, glomerulosclerosis, or lupus, uriniferous tubule disorder caused by nephroblastoma, poisoning, or ischemia, immune-related disorders such as AIDS, thymocyte disorder caused by immune suppressors or carcinostatics, decline of peripheral T cells, or immune deficiency disorders, circulatory organ disorders such as angiostenosis or ischemia; hepatic diseases such as hepatopathy caused by drugs or viral infection, digestive tract disorder, neuropathy such as neurocytic disorders caused by cerebral ischemia, amyotropic lateral sclerosis, neurodegenerative disorders, such as Alzheimer's disease, Huntington's chorea, or Parkinson disease, neuronal developmental disorders (psychosis), etc., rejection accompanied by organ or tissue transplants; damages caused by bacteriotoxin, phytotoxin, or zootoxin; pseudopelade, morphological abnormality; histogenetic deficiency; atrophy of tissues, etc. The above-mentioned diseases are mere examples, and therefore, the agents for treating apoptosis-related disorders of the invention is used not only for these examples, but also other diseases as long as they are related to apoptosis.

The agents for treating apoptosis-related disorders of this invention can be used not only for treating, but also for preventing these disorders.

Although effective concentration and dosage can be determined by experience, about 1 µg to 100 mg/kg can be administered once or several times a day. The determination of the dosage is within the technical scope of a person skilled in the art. The dosage depends on sex, age, body weight or condition of patients. A therapeutically effective amount of MK, suspended in saline, phosphate buffer solution (PBS) etc., can be intravenously administered. Other administration routes include oral, subcutaneous, intramuscular, mucosal, intraperitoneal, or direct administration to a specific organ such as the heart for treating cell death accompanied with myocardial infarction, but are not limited thereto.

### Brief Description of the Drawings

Figure 1 shows the survival rate of Wilma tumor-derived G401 cell lines cultured for 12 and 24 hours in the untreated group (control group), the cisplatin (100 µM) treated group, and the MK (1, 10 or 100 ng/ml) + cisplatin (100 µM) treated groups, indicated by absorbance (OD₅₄₀₋₆₅₅) measured by MTT method.
Figure 2 shows Wilms tumor-derived G401 cell lines stained with Hoechst 33342, which were cultured for 12 and 24 hours, for the untreated group (control group), the cisplatin (100 µM) treated group, and the MK (1, 10 or 100 ng/ ml) + cisplatin (100 µM) treated groups. Panels A, B, and C show 12-hour cultures of the untreated group, the cisplatin treated group, and the MK + cisplatin treated group, respectively. Panels D, E, and F show 24-hour cultures for the untreated group, the cisplatin treated group, and the MK + cisplatin treated group, respectively.
Figure 3 shows the percentage of the number of apoptotic cell deaths with nuclei showing chromatin condensation and nucleic fragmentation, to the total number of cells.

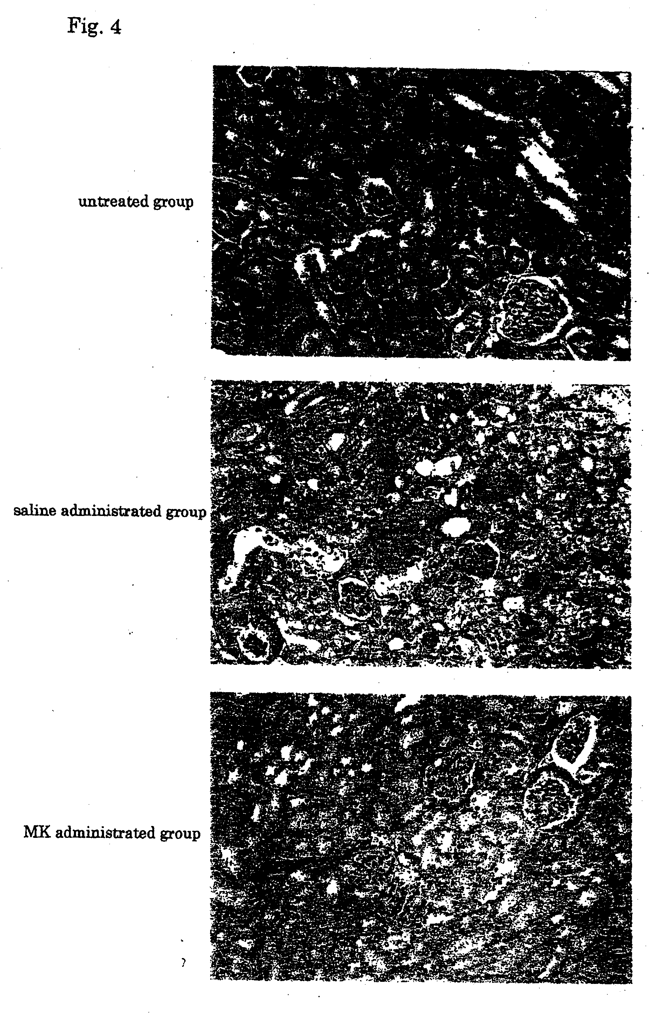

Figure 4 shows the sections of the kidneys from 129/Sv MK knockout mice (heterozygote) (male, 8- to 12-week-old) stained with hematoxylin-eosine. Saline (10 ml) and MK (200 µg) were intraperitoneally administered to the saline administrated group and the MK administrated group in the morning, respectively, and cisplatin (9.3 mg/kg) was intraperitoneally administered to each group in the afternoon (Day 0). From Day 1 to 3, saline (10 ml) and MK (200 µg) were intraperitoneally administered to each group. At Day 4, the kidneys were taken out and embedded with paraffin to prepare sections. The resulting sections were stained with hematoxylin-eosine. As a control, sections were prepared from the untreated wild-type mice and stained with hematoxylin-eosine.
Figure 5 shows the sections embedded with paraffin analyzed by the TUNEL method.
Figure 6 shows the expression of Bcl-2 in Wilms tumor-derived G401 cells analyzed by Western as follows. Six groups were prepared; three cisplatin untreated groups consisting of the MK untreated group, the human MK (100 ng/mL ) treated group, and the mouse MK (100 ng/mL) treated group, and three cisplatin (100 µM) treated groups consisting of the MK untreated group, the human MK (100 ng/mL) treated group, and mouse MK (100 ng/mL) treated group.

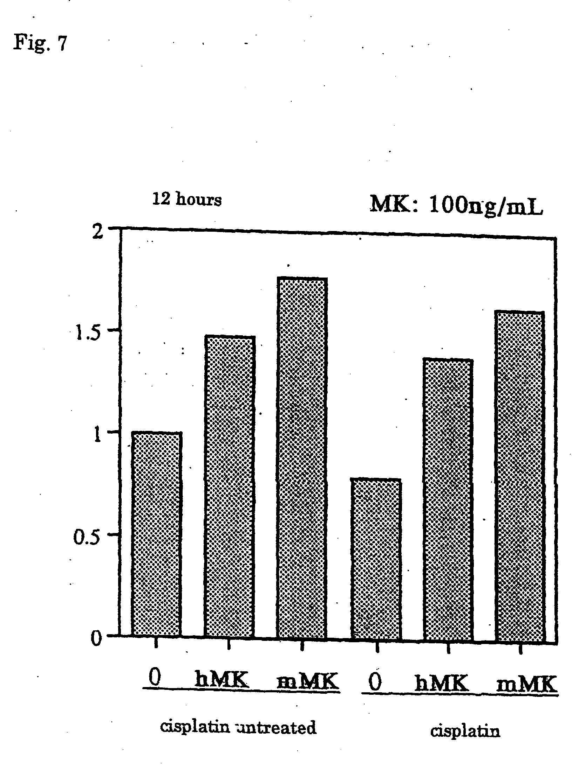

Figure 7 numerically shows the results of Fig. 6.
Figure 8 shows hippocampal region CA1 neurocytes obtained from the Mongolian gerbils to which saline or MK (2 µg each) was injected into the ventricle just before the development of transient forebrain ischemic injury, analyzed by the TUNEL method seven days after the injection. The magnification of the microscope was X 25.
Figure 9 shows the same figure in the magnification of X 100.

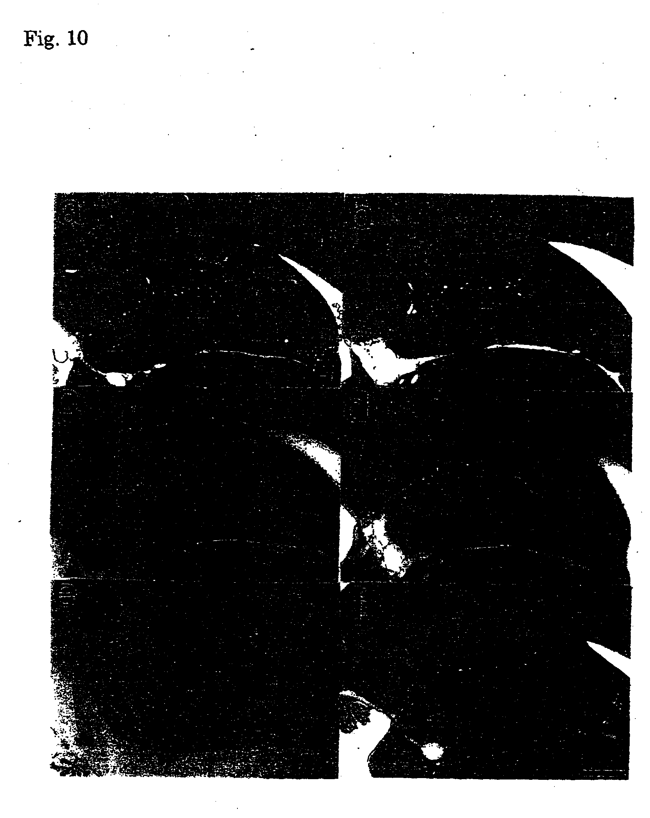

Figure 10 shows the hematoxylin-eosine (HE)-stained sections of hippocampal region CA1 neurocytes obtained from the Mongolian gerbils. The sections were prepared by injecting saline (a), 2 µg MK (c), 1 µgMK (d), 0.5 µg MK (e), or 0.25 µg MK (f) to the ventricle of the Mongolian gerbils just before development of transient forebrain ischemic injury and stained seven days after the injection. The length of the bar represents 0.5 mm. The magnification of the microscope is X 25.

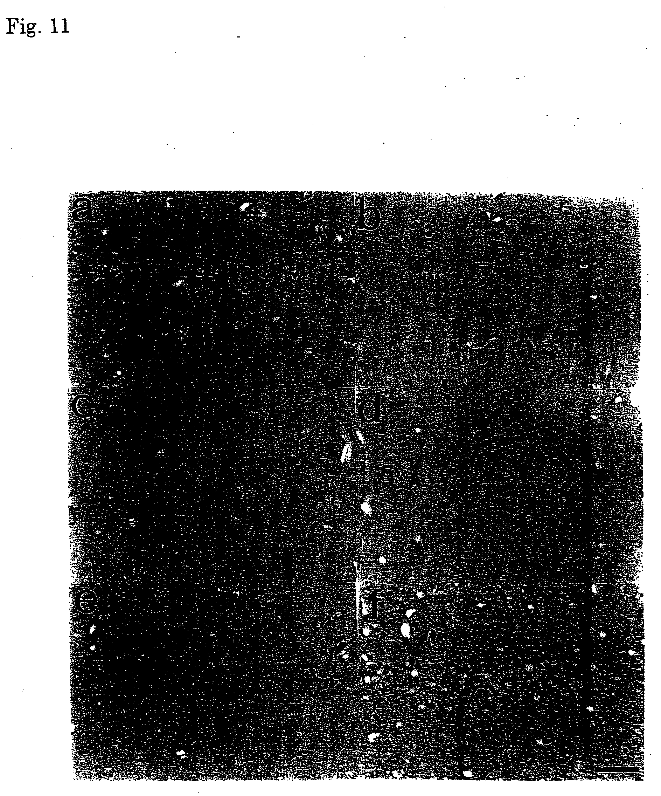

Figure 11 shows the same sections as in Fig. 10 in the magnification of X 100. The length of the bar represents 50 µm.
Figure 12 shows the ratio of the number of nuclei in the surviving neurocytes stained with HE in the hippocampal region CA1 of Fig. 10, to the whole length of CA1 region. n represents the number of animals.

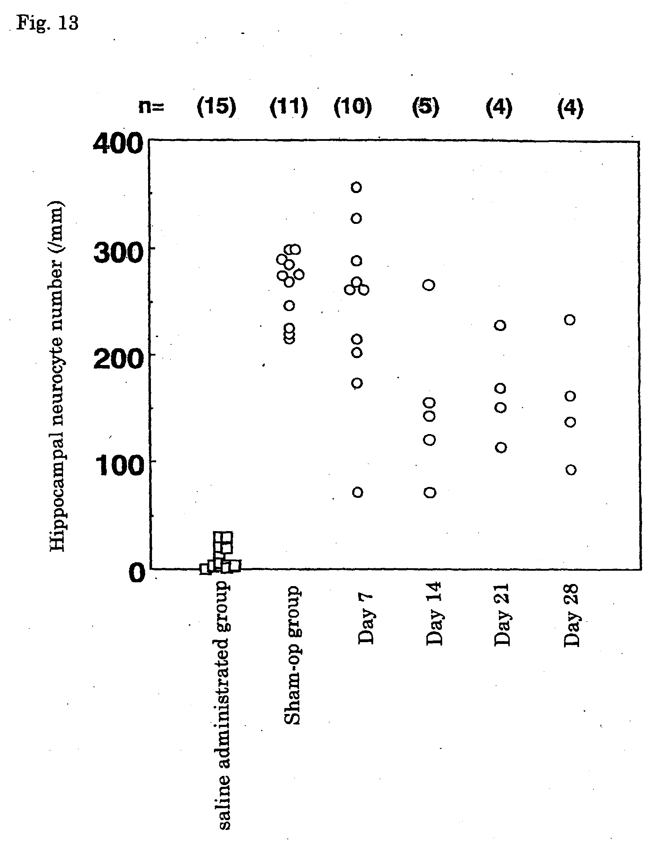

Figure 13 shows the ratio of the number of surviving neurocytes in the hippocampal CA1 region obtained from the Mongolian gerbils, 1, 2, 3, and 4 weeks after the injection of 2 µg MK into ventricle just before development of the transient forebrain ischemic injury, to the whole length of CA1 region.

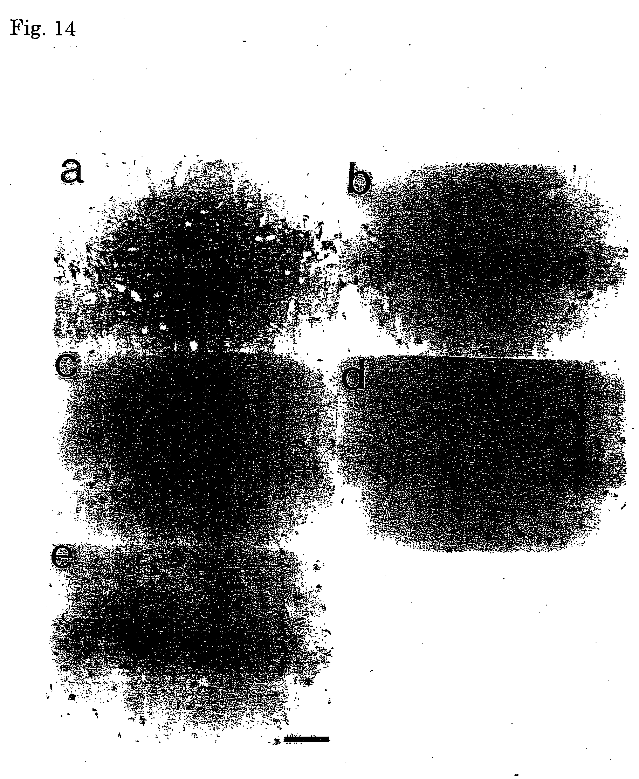

Figure 14 shows the neurocytes in the hippocampal region CA1, analyzed by the TUNEL method. The neurocytes were prepared by injecting saline (a), 2 µg MK x (C), 1 µg MK (d), or 0.5 µg MK (e) to the ventricle of the Mongolian gerbils just before development of transient forebrain injury and analyzed seven days after the injection. The cells with apoptosis were strongly stained. The length of the bar represents 50 µm.

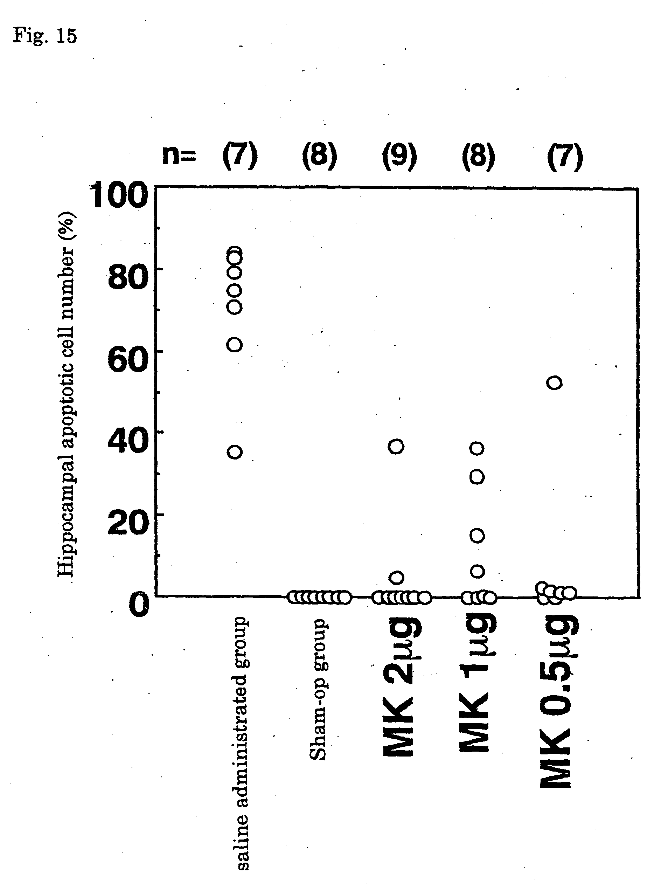

Figure 15 shows the ratio of the number of cells with apoptosis in hippocampal region CA1 to the number of whole cells.
Figure 16 shows the ratio of the number of the surviving neurocytes in hippocampal region CA1 to the length of the CA1 region. The cells were prepared by injecting 2 µg MK into the vertical, 2, 4, 6, 12 and 24 after development of the transient forehead ischemic injury in Mongolian gerbils and the number of the survived cells for each treatment was counted seven days after the injection.

### Best Mode for Carrying out the Invention

The present invention is illustrated in detail below with references to examples, but is not to be construed as being limited thereto.

### Example 1 Preparation of MK

(1) Midkine used for Examples 2 to 7 (1) was prepared by the method of Example 1 of Unexamined Published Japanese Patent Application (JP-A) No. Hei 9-95454 (SEQ ID NO: 3)
(2) Midkine used for Examples 7(2) to 8 was prepared by the following method.

For producing human MK protein, cDNA fragment comprising human MK open reading frame (nucleotide positions 1-432, J. Tsutsui, et al.: Biochem. Biophys. Res. Commun. Vol. 176, 792-797, 1991) was inserted into the yeast expression vector pPIC9 (Invitrogen). This recombinant plasmid was transfected into yeast (*Pichia pasrotis* GS115; Research Corporation Technologies), and the desired clones were selected with histidine and G418. Human MK protein secreted by yeast into the culture medium was purified by employing the following column chromatography in the order below.
1. SP Steamlines (Pharmacia; adsorption and wash with 20 mM pH 5.5 acetate buffer, elution with 20 mM pH 3.5 acetate buffer containing NaCl)
2. Sulfated Cellulofine (Seikagaku Kogyo, Japan; adsorption with 10 mM pH 7.2 phosphate buffer, wash with buffer containing 0.7 M NaCl, elution with buffer containing 2.0 M NaCl)
3. Superdex 75 pg (Pharmacia; gel filtration with saline)
4. Poly Sulfoethyl A (Poly LC Co.; adsorption with 20 mM buffer containing 0.6 M NaCl, wash with buffer containing 0.88 M NaCl, elution with buffer containing 2M NaCl).
5. Superdex 75 pg (Pharmacia; gel filtration with saline).

Midkine preparation was dialyzed against saline, and 0.1 ml aliquots of the purified proteins were stored at -80 °C. The protein was used immediately after it was thawed so as not to refreeze and rethaw it. The activity of purified MK proteins was detected by using as an index the activity of MK for promoting the survival of embryonic neurons (M. Michikawa, et al.: J. Neurosci. Res. Vole 35, 530-539, 1993).

### Example 2 Inhibitory effects on apoptosis induced by a carcinostatic (MTT method)

Apoptosis was induced in G401 cell lines derived from Wilms tumor, infantile renal cancer, by a carcinostatic to examine the apoptosis-inhibitory effect of MK by the MTT method.

Five groups were prepared; the untreated group, the cisplatin (100 µM) treated group, and the MK (1, 10 or 100 ng/mL) + cisplatin treated groups.

G401 cells were suspended in the Dulbecco Modified Eagle Medium containing 10% fetal bovine serum (FBS) (10% FBS/DMEM) and seeded into a 96-well plate at 5000 cells/well. The plate was incubated in a CO₂ incubator (37°C, 5% CO₂; the same shall apply hereinbelow) so that the cells could attach to the plate.

The cells were washed with DMEM containing 0.1% FBS (0.1% FBS/DMEM) twice (hereafter, 0.1% FBS/DMEM was used for washing unless otherwise indicated), and cultured in 0.1% FBS/DMEM overnight. After culturing, MK was added to the MK treated groups at 1, 10 or 100 ng/ml. Each group was cultured for 6 hours. Six µl of 0.5 mg/ml cisplatin (100 µM) (Bristol-Myers Squib Company; Tokyo) was added to the cisplatin treated groups. Each group was cultured in 0.1% FBS/DMEM for 2 hours. The cells were then washed three times and, MK was added to the MK treated groups to 1, 10, or 100 ng/ml. Each group was cultured in 0.1% FBS/DMEM for 12 hours or 24 hours.

Fifteen µl of MTT reagent (5 mg/ml) (Wako Pure Chemical Laboratories; Osaka) were added to each well of the above-mentioned five groups and cultured for 4 hours. Then 100 µl of 10 mM hydrogen chloride containing 20% SDS were added to each well and kept for 24 hours at room temperature. Absorbance (OD₅₄₀₋₆₅₅) in each well was measured using a microplate reader. The results are shown in Fig. 1.

Among the 12 hour-cultured groups, the MK (1, 10 or 100 ng/mL) + cisplatin (100 µM) treated groups showed increased numbers of the surviving cells in an MK concentration-dependent manner, compared with the cisplatin treated group. This result reveals that MK inhibited cell death caused by the carcinostatic.

### Example 3 Inhibitory effect on apoptosis induced by carcinostatic (Hoechst staining method)

Five groups were prepared; the untreated group, the cisplatin (100 µM) treated group, the MK (1, 10, or 100 ng/mL) + cisplatin (100 µM) treated groups.

G401 cells (2 X 10⁴) were seeded on a 3.5 cm-dish and cultured in 10% FBS/DMEM overnight. 10 % FBS/DMEM was added to each dish and further cultured for 24 to 48 hours. The cells were washed twice and cultured in 0.1% FBS/DMEM overnight. MK was added to the MK treatment groups to 1, 10, or 100 ng/ml and cultured in 0.1% FBS/DMEM for 6 hours. Cisplatin was added to the cisplatin treatment groups to 100 µM and cultured in 0.1% FBS/DMEM for 2 hours. After culturing, the cells were washed three times. Then, human MK was added to the MK treatment groups to 1, 10, or 100 ng/ml and cultured in 0.1% FBS/DMEM for 12 or 24 hours.

The cells were fixed with a fixative (methanol:acetic acid = 3:1) at room temperature for 10 min, and dried at room temperature for 30 min. Hoechst 33342 (ICN Biomedicals; Ohio, USA) was added thereto to stain the cells at room temperature for 10 to 30 min. The cells were washed with water three times, wind-dried, and mounted onto a fluorescent microscope (Olympus, BX60) and photographed. Figures 2A, 2B, and 2C show the untreated group, the cisplatin (100 µM) treated group, and the MK (100 ng/mL) + cisplatin (100 µM) treated group, which were all cultured for 12 hours. Figures 2D, 2E, and 2F show the untreated group, the cisplatin (100 µM) treated group, and the MK (100 ng/mL) + cisplatin (100 µM) treated group, which were all cultured for 24 hours. The numbers of cells containing the Hoechst 33342-stained nuclei with condensed chromatin and fragmented nuclei were remarkably decreased in C and F, compared to those in B and E.

Figure 3 shows the ratio of the number of cells that died from apoptosis and contained nuclei with condensed chromatin and the fragmented nuclei, to the total number of cells with the nuclei stained by Hoechst 33342. Frequency of apoptotic cell death was decreased in a MK concentration-dependent fasion in the MK (1, 10, or 100 ng/ml) + cisplatin (100 µM) treated groups cultured for both 12 and 24 hours, compared with that in the cisplatin (100 µM) treated group. In the 24-hour culture, the number of live cells also increased compared to that in the 12-hour culture.

### Example 4 Determination of DNA content in apoptosis-induced neurocytes

The culture flasks were coated with MK 100 mg/ml or MK 10 µg/ml. The flasks without a MK-coating (control) were also prepared.

NG 108 cell lines, hybrid cells of mouse neuroblastoma C1300 and glioma (Nelson P. G. et al.: Brain Res., 147: 245, 1978), were cultured in a Falcon 3110 cell culture flask (75 cm², BECTON DICKINSON) containing 10% FBS/DMEM until the cells were almost confluent. After culturing, the cells were exposed to ultraviolet light for several hours (UV radiation amount: 60-100 J/m²) to induce apoptosis.

The cells were collected by centrifugation (4°C, 1000 rpm, 10 min), and washed with 10 to 12 ml of sample buffer [1 g glucose/1 L PBS (-); filtered through a 0.22 µm filter] twice. The cells were adjusted to 1 X 10⁶ to 3 X 10⁶ cells/ml with the sample buffer, and 1 ml of them was centrifuged (4°C, 1000 rpm, 10 min) in a centrifuge tube (FALCON; 15 X 75 mm). The supernatant was discarded and the pellet was vigorously vortexed for 10 sec. Ethanol (70 %) cooled with ice (1 ml) was added dropwise thereto and fixed at 4°C overnight.

The cells fixed overnight were briefly vortexed and centrifuged (3000 rpm, 5 min). 70% Ethanol in the supernatant was discarded to 0.2 ml or less. The centrifuge tubes containing the cells were slowly vortexed, and 1 ml of propidium iodide (PI) stain was added thereto. The PI stain contained 0.5 ml of 20 X PI solution, 0.1 ml of 100 X RNase A solution, and 10 ml of the sample buffer. Twenty-fold dilution of PI was obtained by preparing 1 mg/ml of PI solution (SIGMA), filtering it through a 0.22 µm filter, and storing it at 4°C in the dark. RNase A solution (100 X) was prepared by adjusting RIBONUCLEASE A (Type I-A) (SIGMA) (10000 U/ml) to 125 mg/ml (80 U/mg).

The cells were lightly shaken, incubated for 30 min or longer at room temperature, and measured by flow cytometry. The results are shown in Table 1. In the presence of MK, the ratio of the cells at G1 phase increased by about tenfold, compared with the control group, while the ratio of the cells at S phase decreased one eighth to one forth. These results indicate that MK can suppress cellular apoptosis induced by ultraviolet light or radiation.

**Table 1**

| Ratio of cells at each cell cycle (%) | | | |
|---|---|---|---|
| | Control group | MK 100 ng/ml | MK 10 µg/ml |
| G1 | 7 | 75 | 67 |
| S | 84 | 19 | 9 |
| G2/M | 9 | 5 | 24 |
| Total | 100 | 100 | 100 |

### Example 5 Inhibitory effect on apoptosis in renal uriniferous cells

Heterozygous 129/Sv knockout mice (male, 8- to 12-week old) in which parts of exons 2 and 3 of the MK gene were destroyed (Biochemistry 7, 1996, Volume 68, pp. 1239, 4-p-1244) were divided into two groups; the saline administration group and the MK administration groups (14 individuals each). As a control group, the untreated wild-type mice (four individuals) were prepared. Dosage of MK was 200 µg/kg and that of saline was 10 ml/kg.

MK or saline was intraperitoneally administered to the mice in two groups in the morning and cisplatin 9.3 mg/kg was intraperitoneally administered to the mice in two groups in the afternoon of the same day (this day is Day 0).

In the morning of Day 1, MK or saline was interperitoneally administered to each mouse in the same manner as on Day 1.

On Day 2, four mice were selected from each group (the untreated wild-type mice group and the two treated groups) to collect urine and whole blood. Clots in the collected blood were retracted for 1 hour at 37°C, and centrifuged (4°C, 8000 rpm, 10 min) to separate the serum. The serum was stored at -20°C until the experiment. Kidneys were excised from each mouse and fixed with buffer formalin (for 2 to 4 days) and embedded with paraffin using an automatic embedding machine (SAKURA, ETP-120A). The kidneys embedded with paraffin were cut to adjust to 4 µm using a microtome (ERMA OPTICAL WORKS LTD, No. 1061). As to the rest of the mice in the two groups, MK or saline was intraperitoneally administered in the same manner as on Day 1.

On Day 3, six mice were selected from each of the two treated groups to collect urine and whole blood in the same manner as on Day 2. Kidneys were excised from each mouse and embedded with paraffin. As to the rest mice in the two groups, MK or saline was intraperitoneally administered in the same manner as on Day 2.

On Day 4, the last day, urine and whole blood were collected from four individuals in each of the two groups. The kidneys of each mouse were excised and embedded with paraffin.

Blood urea nitrogen (BUN) and serum creatinine frequently used as indexes of renal dysfunction in day-to-day diagnosis were measured and proteins in urine were simply quantified. The paraffin sections of the kidneys were stained with hematoxylin-eosine (HE) (Fig. 4). The effect of MK for relieving renal dysfunction caused by cisplatin was observed in the MK administrated group. The result implies that MK can suppress apoptosis in renal uriniferous tubule cells. Subsequently, the following experiment was performed.

On Day 4, DNA fragmentation in the kidney paraffin sections representating strong damage was analyzed by the TUNEL method (In situ Apoptosis Detection Kit; TaKaRa). The results are shown in Fig. 5. Some nuclei were TUNEL reaction-positive in the saline administrated group, while the number of TUNEL positive cells significantly decreased in the MK administrated group. No TUNEL positive nuclei were observed in the untreated wild-type group. These results indicate that MK suppresses cisplatin -induced apoptosis in renal uriniferous tubule cells.

### Example 6 Effect for enhancing Bcl-2 expression

The effect of MK on the expression of the Bcl-2 gene was examined. Six groups were prepared; the untreated group, the cisplatin (100 µM) treated group, the human MK (100 ng/ml) treated group, the human MK (100 ng/ml) + cisplatin (100 µM) group, the mouse MK (100 ng/ml) treated group, and the mouse MK (100 ng/ml) + cisplatin (100 µM) treated group.

G401 cells were seeded on a 3.5 cm-dish and cultured in 10% FBS/DMEM until the cells became almost confluent. The cells were washed twice and cultured in 0.1% FBS/DMEM overnight. Then, human MK or mouse MK (JP-A No. Hei 9-196293) was added to the MK treatment groups at 100 ng/ml and cultured for six hours. Cisplatin was added to the cisplatin treatment group to 100 µM, and cultured for 2 hours. After culturing, the cells were washed with 0.1% FBS/DMEM three times, cultured in 0.1% FBS/DMEM for 10 hours, and washed with PBS three times.

Two hundred microliters of buffer [1% Triton X-100, 150 mM NaCl, 10mM Tris (pH 7.4), 1.0 mM EGTA, 0.5% NP-40, 1 mM PMSF, 0.1 µg/ml apiotinin, 40 nM Iewpeptin] were added to each dish and kept on ice for 20 min. The cells in each dish were detached by a scraper, and mixed 8 to 10 times using a syringe with a 26-gauge needle. The cells of each dish were centrifuged in a centrifuge tube at 4°C at 12000 rpm for 30 min. The precipitated proteins were quantified (BCA Kit; PIERCE). The precipitate prepared from each dish (50 µg) was analyzed by Western blotting using anti-Bcl-2 monoclonal antibody (500 X) (Dako; Denmark) and anti-mouse HRP antibody (Jackson Immuno Research) (Fig. 6). The band in each lane is numerically shown (Fig. 7).

Figures 6 and 7 show that human MK and mouse MK enhanced the expression of Bcl-2 in the cultured cells. A similar enhancing effect was also observed for cells treated with a carcinostatic (Fig. 7). The concentration showing the enhancing effect was about 10 ng/ml or higher when using these cells (data not shown).

### Example 7 Apoptosis-inhibitory effect in vivo (administration before ischemic damage)

(1) Six to 16 male Mongolian gerbils (6- to 8-week old, body weight: 60 to 80 g) for each group were housed in "HONEY MATIC M-3" (KIMURA MEDICAL INSTRUMENT LTD.), an anesthetic delivery device for Fluothane, and the container was filled with an appropriate amount of inhalation anesthetic "Fluothane" (halothane according to Japanese Phamacopeia). The anesthetized animals were fixed on an operating table equipped with an injection holder (NARISHINGE SCEITIFIC INSTRUMENT LAB.; TYPE SR-5N, No. 97024). After the head was subjected to midline incision, a hole for inserting a syringe of an appropriate size was made by a dental drill at a site2 mm away from the bregma towards the left eyeball direction. Through this hole, 2 µl of 0.25 mg/ml, 0.5 mg/ml or 1 mg/ml MK solutions (0.5 µg, 1.0 µg 2.0 µg ) (in physiological saline) were seperately injected into the ventricle with a microsyringe (HAMILTON MICROLITER #701). As control groups, a saline injected group and the sham operated (Sham-op) group were prepared. After injection into the ventricle, the mice were left for 4 min and the operated site was sutured. The chest was subjected to midline incision to expose both right and left common carotid arteries. The both arteries were ligated with two pieces of Sugita brain aneurysm clip (standard type; MIZUHO) to stop blood flow for 5 min, and the blood was circulated again. During ischemic loading, brain temperature and body temperature were maintained constant (37 ± 0.2°C). Each mouse was distinguished and housed in a nursery cage after coming out of anesthesia to breed, allowing the animals to freely intake water and food. After one week, the mice were fixed while perfusing with saline containing 0.2% heparin (Novo Heparin Injection 100; Japan Hoechet Marion Russell LTD) and 4% paraformaldehyde solution, and beheaded. The brain was excised using scissors, and immersed in 4% paraformaldehyde fixative for one day. The rear portion from 2 mm ahead of the bregma was divided into three using a double-edged razor (Feather). These sections were further fixed for 24 hours. Tissues containing dorsal hippocampus were dehydrated and penetrated, and embedded with paraffin.
   A 5 µm section, from 0.5 to 1.0 mm from the tip of the hippocampus or from 1.4 to 1.9 mm behind the sagittal suture was prepared from this paraffin block and observed by the TUNEL method (In situ Apoptosis Detection Kit, TaKaRa). The results are shown in Fig. 8 (x 25) and Fig. 9 (x 100). Figure 8 shows numerous cells containing TUNEL reaction positive nuclei in the hippocampal neuronal region CA1 observed only in the saline administrated group. Figure 9 also shows cells containing TUNEL reaction positive nuclei observed only in the saline administration group, and also shows many atrophied neurocytes. In the Sham-op group or the MK administrated group, such neurocytes were not observed. These results revealed that MK can protect neurocytes from apoptosis induced by ischemic stress.
(2) By following the method described in (1), MK solution (0.063 µg, 0.125 µg, 0.25 µg, 0.5 µg, 1 µg, or 2 µg), was injected into the ventricle just before development of ischemic injury to examine protective effects of MK on delayed neuronal death. Seven days after the injury, the CA1 region were stained with hematoxylin-eosine. Micrographs in low magnification (x 25) are shown in Fig. 10 (Fig. 10a: saline, Fig. 10b: Sham-op, Fig. 10c: MX 2 µg, Fig. 10d: MK 1 µg, Fig. 10e: MK 0.5 µg, Fig. 10f: MK 0.25 µg), and those in high magnification (x 100) in Fig. 11 (Fig. 11a: saline, Fig. 11b: Sham-op, Fig. 11c: MK 2 µg, Fig. 11d: MK 1 vg, Fig. 11e: MK 0.5 vg, Fig. 11f: MK 0.25 µg). No morphological difference was observed in the saline administrated group (Fig. 10a), the control Sham-op group (Fig. 10b), and the various MK concentration-administration groups (Fig.s 10c to f). The number of neurocytes in the saline administered group remarkably decreased, compared to the Sham-op group (Fig. 10b) or the 0.5 to 2 µg MK administrated groups (Fig. 10c to e). In high magnification, the deterioration of atrophied nuclei was observed in the saline group (Fig. 11a), while survived circle nuclei were observed in the Sham-op group (Fig. 11b) or 0.5 to 2 µg MK administrated groups (Fig. 11c to e). Deteriorated nuclei were observed in the 0.25 µg MK administrated group (Fig. 11f, 1.0 mM EDTA).

For quantitative evaluation, the numbers of surviving nuclei contained in the CA1 region per unit length of CA1 were obtained (Fig. 12). The values were 18 ± 25/mm (n = 15, mean ± standard deviation) for the saline administration group and 265 ± 38 /mm (n = 23) for the Sham-op group. For the 0.5, 1, or 2 µg MK administration groups, the values were 217 ± 109 (n = 7), 228 ± 84 (n = 8), and 243 ± 82 (n = 10), respectively. The values decreased when MK concentration was low (Fig. 12). There was a significant difference between the saline administrated group and MK administrated (0.5 to 2 µg MK) groups (ANOVA and post-hoc Fisher's PLSD teat, p<0.0001).

Whether the effect of MK administration (2 µg) continues was examined. The numbers of neurocytes were 263 ± 31 cells/mm (n = 11) in the sham-op group and 10 ± 10 cells/mm (n = 15) in the saline administrated group in which the animals were beheaded seven days after the ischemic injury. The numbers of neurocytes in the Mongolian gerbils that was given 2 µg MK again and beheaded 1, 2, 3, or 4 weeks after ischemia are as follows:
One week after: 219 ± 74 (n = 10)
Two week after: 152 ± 72 (n = 5)
Three week after: 185 ± 83 (n = 4)
Four week after: 157 ± 60 (n = 4)

Comparison by ANOVA and post-hoc Fisher's PLSD test (p<0.0001) indicated that the survival rate of neurocytes in any test group was significantly much higher than that in the saline administrated group. The number of neurocytes were decreased at two weeks compared to the number at one week, but this number did not decrease thereafter even after four weeks (Fig. 13). Therefore, MK prevents delayed neuronal death rather than delaying it.

Because delayed neuronal death in hippocampal region CA1is thought to be caused by apoptosis, DNA fragmentation by apoptosis was examined by staining the sections by the TUNEL method. MK was administered just before ligation and the effect was evaluated seven days after the ischemic injury. Numerous strongly stained nuclei were observed in the saline administrated group (Fig. 14b), while no stained nuclei were observed in the Sham-op group (Fig. 14b). Few stained nuclei were observed in the 0.5 to 2 µg MK administrated groups (Fig.s 14c to e). The quantitative evaluation was conducted by measuring the number of nuclei showing apoptosis positive reaction against the total number of cells (Fig. 15). The values were 69.7 ± 17.0 (n = 7) for the saline administrated group, and 0.0 ± 0.0 (n = 8) for the Sham-op group. For 0.5, 1, and 2 µg MK administrated groups, the values were 7.8 ± 19.8 (n = 7), 11.0 ± 14.7 (n = 8), and 4.6 ± 12.2 (n = 7), respectively. There was a statistically significant difference between the saline administrated group and the MK (0.5 to 2 µg) administrateed groups (ANOVA and post-hoc Fisher's PLSD test, p<0.0001). From these results, MK was demonstrated to suppress delayed neuronal death caused by apoptosis.

### Example 8 Apoptosis-inhibitory effect in vivo (MK administration after the ischemic injury)

MK (2.0 µg) was injected into the ventricle of a Mongolian gerbil transient forebrain ischemia model 2, 4, 6, 12, or 24 hours after the ischemic injury. The numbers of surviving hippocampal neurocytes were counted seven days after the ischemic injury (Fig. 16). When MK was administered at 2 hours after the injury, the number of surviving neurocytes decreased, compared to the Sham-op group, while it was considerably higher than the saline administrated group. Survival rate also increased even when MK was administered 24 hours after the injury (Fig. 16).

The numbers of surviving hippocampal neurocytes in the groups administered 2, 4, 6, 12, or 24 hours after the ligation were 120 ± 36 (n = 4), 90 ± 23 (n = 6), 80 ± 13 (n = 6), 86 ± 34 (n = 6), and 93 ± 22 (n = 4) cells/mm, respectively (Fig. 16). These values were significantly higher in the saline administrated group (p<0.05).

### Industrial Applicability

The present inventors discovered that proteins belonging to the MK family can delay or suppress apoptosis induced by various stimuli such as carcinostatics, ultraviolet light and radiation, ischemic stresses, etc. Based on this finding, the present invention provides novel agents for treating or preventing various diseases attributed to apoptosis, such as cerebropathy, cardiopathy, nephropathy, neuropathy, or hepatopathy, etc., comprising a protein belonging to the MK family as an effective ingredient.

## Claims

1. An agent for suppressing apoptosis, comprising a protein belonging to the MK family as an effective ingredient.

2. The agent for suppressing apoptosis of claim 1, wherein the protein belonging to the MK family is midkine.

3. An agent for treating or preventing apoptosis-related diseases, comprising a protein belonging to the MK family as an effective ingredient.

4. The agent for treating or preventing apoptosis-related diseases of claim 3, wherein the apoptosis-related disease is cardiopathy.

5. The agent for treating or preventing apoptosis-related diseases of claim 3, wherein the apoptosis-related disease is nephropathy.

6. The agent for treating or preventing apoptosis-related diseases of claim 3, wherein the apoptosis-related disease is hepatopathy.

7. The agent for treating or preventing apoptosis-related diseases of claim 3, wherein the apoptosis-related disease is a neurodegenerative disease.

8. The agent for treating or preventing apoptosis-related diseases of any one of claims 3 to 7, wherein the protein belonging to the MK family is midkine.

9. A Bcl-2 enhancer comprising a protein belonging to the MK family as an effective ingredient.
